# EUROPEAN PATENT SPECIFICATION

(11) **EP 4 126 201 B1**
(45) Date of publication and mention of the grant of the patent: **31.01.2024**
(21) Application number: 20803264.9
(22) Date of filing: 22.10.2020
(51) Int. Cl.: A61N 1/362, A61N 1/365, A61N 1/39, A61B 5/1455, A61B 5/08, A61B 5/091

(54) **CARDIAC CONTRACTILITY MODULATION IN ASSOCIATION WITH RESPIRATION**
HERZKONTRAKTILITÄTSMODULATION IN VERBINDUNG MIT DER ATMUNG
MODULATION DE LA CONTRACTILITÉ CARDIAQUE EN ASSOCIATION AVEC LA RESPIRATION

(30) Priority: 29.03.2020 US 202063001343 P
(43) Date of publication of application: 08.02.2023
(73) Proprietor: Impulse Dynamics NV, Willemstad (CW)
(72) Inventor: PRUTCHI, David, Voorhees, New Jersey 08043 (US); BEN DAVID, Tamir, 6998827 Tel-Aviv (IL)
(74) Representative: Puchberger & Partner Patentanwälte
(86) International application number: PCT/IB2020/059944
(87) International publication number: WO 2021/198755

(56) References cited:
- EP-A1- 0 910 429
- EP-A1- 2 659 931
- US-A1- 2004 127 804
- US-A1- 2005 039 745
- US-A1- 2017 348 524

## Description

### FIELD AND BACKGROUND OF THE INVENTION

The present invention, which is defined in the appended claims, in some embodiments thereof, relates to cardiac contractility modulation cardiac contractility modulation treatment, and, more particularly, but not exclusively, to applying of cardiac contractility modulation stimulation in association with a respiratory stage or condition. EP0910429B1 relates to cardiac muscular control, in particular control using non-excitatory electrical signals.

### SUMMARY OF THE INVENTION

Any methods disclosed hereinafter are not claimed, and are mentioned for illustrative purposes only.

According to an aspect of some embodiments there is provided a method for delivering cardiac contractility modulation stimulation to the heart via an implanted cardiac device, comprising: determining at least one respiration parameter; and setting one or more parameters of cardiac contractility modulation stimulation according to the at least one respiration parameter.

In some embodiments, the at least one respiration parameter is selected from the group of: a respiration rate, a relative timing of exhalation and/or inhalation, a duration of exhalation and/or inhalation.

In some embodiments, setting one or more parameters of cardiac contractility modulation stimulation comprises setting timing for applying the cardiac contractility modulation stimulation in accordance with a relative anatomical space between the heart and the diaphragm and/or a relative anatomical space between the heart and the phrenic nerve, as correlated with the respiration cycle.

In some embodiments, the method comprises synchronizing timing for applying the cardiac contractility modulation stimulation with timing in which the diaphragm is farthest from the heart and/or with timing in which the phrenic nerve is farthest from the heart.

In some embodiments, setting one or more parameters of cardiac contractility modulation stimulation comprises setting timing for applying the cardiac contractility modulation stimulation, the timing being during an absolute refractory period of the cardiac cycle.

In some embodiments, determining comprises measuring the at least one respiration parameter using one or more sensors.

In some embodiments, the one or more sensors are selected from the group of: an acoustic sensor, a pulse oximeter, a pneumograph, a capnograph.

In some embodiments, the one or more sensors include an intra-cardiac electrode which records ECG, and wherein the determining at least one respiration parameter is based on the ECG recording.

In some embodiments, setting one or more parameters of cardiac contractility modulation stimulation comprises setting a current intensity of the cardiac contractility modulation stimulation at the highest level which does not cause pain.

In some embodiments, setting one or more parameters of cardiac contractility modulation stimulation comprises setting a rate for applying multiple cardiac contractility modulation stimulations.

In some embodiments, the method comprises increasing the rate of cardiac contractility modulation stimulations when a respiration rate rises, and decreasing the rate of cardiac contractility modulation stimulations when a respiration rate decreases.

In some embodiments, the cardiac device is implanted in a patient diagnosed with heart failure.

In some embodiments, setting one or more parameters of cardiac contractility modulation stimulation comprises selecting one or more intervals of the respiratory cycle for applying of cardiac contractility modulation stimulations.

In some embodiments, selecting comprises testing during which time interval(s) the applying does not cause or only least causes sensation to the patient.

In some embodiments, the method comprises selecting a time interval during the inhalation period for applying of cardiac contractility modulation stimulation.

In some embodiments, the method comprises selecting a time interval during the exhalation period for applying of cardiac contractility modulation stimulation.

In some embodiments, the method comprises recording an ECG of the patient and selecting a time for applying of cardiac contractility modulation stimulation when an amplitude of the R wave of a current cardiac cycle is higher than an average R wave amplitude calculated based on at least 5 previous cardiac cycles.

In some embodiments, the method comprises recording an ECG of the patient and selecting a time for applying of cardiac contractility modulation stimulation when an amplitude of the R wave of a current cardiac cycle is lower than an average R wave amplitude calculated based on at least 5 previous cardiac cycles.

In some embodiments, the method comprises recording an ECG of the patient and selecting a time for applying of cardiac contractility modulation stimulation when the RR interval of a current cardiac cycle is longer than an average RR interval calculated based on at least 5 previous cardiac cycles.

In some embodiments, the method comprises recording an ECG of the patient and selecting a time for applying of cardiac contractility modulation stimulation when the RR interval of a current cardiac cycle is shorter than an average RR interval calculated based on at least 5 previous cardiac cycles.

In some embodiments, determining at least one respiration parameter comprises tracking the respiratory cycle to detect a dyspnea episode and apply cardiac contractility modulation stimulation in response.

In some embodiments, the method comprises assessing cardiac output and/or respiratory output over time and timing the applying of cardiac contractility modulation stimulation accordingly.

In some embodiments, the method comprises assessing if the patient has sensed the cardiac contractility modulation stimulation during a selected time interval of the respiratory cycle, and selecting a different time interval of the respiratory cycle for applying of the cardiac contractility modulation stimulation if the stimulation was sensed.

According to an aspect of some embodiments there is provided a system comprising:
an implantable cardiac device comprising: at least one lead comprising one or more electrodes for applying cardiac contractility modulation stimulation to the heart; and
circuitry for controlling and activating the leads, the circuitry programmed to set at least one of timing and an intensity of the cardiac contractility modulation stimulation current according to a detected respiratory rate.

In some embodiments, the system comprises a sensor configured for detecting the respiratory rate.

In some embodiments, the sensor is selected from the group of: an acoustic sensor, a pulse oximeter, a pneumograph, a capnograph.

In some embodiments, the system comprises an intra-cardiac electrode for recording ECG; wherein the respiratory rate is determined according to the recorded ECG.

In some embodiments, the circuitry comprises a controller programmed to increase a rate of cardiac contractility modulation stimulations upon a detected rise in the respiratory rate and to decrease the rate of cardiac contractility modulation stimulations upon a decrease in the respiratory rate.

In some embodiments, the device comprises at least one lead comprising one or more electrodes for applying an electrical stimulation to the diaphragm and/or to the phrenic nerve.

In some embodiments, the circuitry comprises a controller programmed to control parameters of the stimulation applied to the diaphragm and/or phrenic nerves, the parameters comprising timing for applying the stimulation and an intensity of the stimulation current.

According to an aspect of some embodiments there is provided a method for timing delivery of cardiac contractility modulation stimulation to the heart, comprising: applying cardiac contractility modulation stimulation to the heart; determining if the cardiac contractility modulation stimulation caused pain and/or sensation to the patient; assessing if the pain and/or sensation were associated with stimulation of the diaphragm, Phrenic nerve and/or its branches; and setting one or more parameters for applying additional cardiac contractility modulation stimulation in accordance with the respiration cycle of the patient.

In some embodiments, setting one or more parameters comprises setting timing for applying of the additional cardiac contractility modulation stimulation, the timing including a time interval of the respiration cycle during which applying of the cardiac contractility modulation stimulation caused least pain or sensation to the patient.

In some embodiments, cardiac contractility modulation stimulation is applied during a refractory period of the cardiac cycle.

According to an aspect of some embodiments there is provided a system comprising:
one or more sensors for detecting parameters of a respiratory cycle in a patient; and
an implantable cardiac device comprising: at least one lead comprising one or more electrodes for applying cardiac contractility modulation stimulation to the heart; and circuitry for controlling and activating the leads, the circuitry programmed to set parameters of the cardiac contractility modulation stimulation according to the parameters of the respiratory cycle detected by the one or more sensors.

In some embodiments, the one or more sensors are configured to detect parameters selected from the group of: respiration rate, relative timing of inhalation and exhalation, ECG of the heart.

In some embodiments, the parameters of the cardiac contractility modulation stimulation including timing of the cardiac contractility modulation stimulation, an intensity of the cardiac contractility modulation stimulation current.

According to an aspect of some embodiments there is provided a method for timing delivery of cardiac contractility modulation stimulation to the heart, comprising: tracking the respiration cycle in a patient; identifying one or more time periods of the respiration cycle during which the diaphragm is non or least excitatory and/or non or least contractible; and applying cardiac contractility modulation stimulation to the heart during the identified one or more time periods.

In some embodiments, applying is during a refractory period of the cardiac cycle.

According to an aspect of some embodiments there is provided a method of operating an implanted cardiac device configured for delivery of cardiac contractility modulation stimulation to the heart, comprising:
measuring at least one respiration parameter using one or more sensors; and
automatically setting, at a controller of the implanted cardiac device, one or more parameters of cardiac contractility modulation stimulation according to the at least one measured respiration parameter.

In some embodiments, the at least one respiration parameter is selected from the group of: a respiration rate, a relative timing of exhalation and/or inhalation, a duration of exhalation and/or inhalation.

In some embodiments, automatically setting one or more parameters of cardiac contractility modulation stimulation comprises setting timing for applying the cardiac contractility modulation stimulation in accordance with a relative anatomical space between the heart and the diaphragm and/or a relative anatomical space between the heart and the phrenic nerve, as correlated with the respiration cycle.

In some embodiments, the method comprises synchronizing timing for applying the cardiac contractility modulation stimulation with timing in which the diaphragm is farthest from the heart and/or with timing in which the phrenic nerve is farthest from the heart.

In some embodiments, setting one or more parameters of cardiac contractility modulation stimulation comprises setting timing for applying the cardiac contractility modulation stimulation, the timing being during an absolute refractory period of the cardiac cycle.

In some embodiments, the one or more sensors are selected from the group of: an acoustic sensor, a pulse oximeter, a pneumograph, a capnograph.

In some embodiments, the one or more sensors include an intra-cardiac electrode which records ECG, and wherein the determining at least one respiration parameter is based on the ECG recording.

In some embodiments, automatically setting one or more parameters of cardiac contractility modulation stimulation comprises setting a current intensity of the cardiac contractility modulation stimulation at the highest level which does not cause pain.

In some embodiments, automatically setting one or more parameters of cardiac contractility modulation stimulation comprises setting a rate for applying multiple cardiac contractility modulation stimulations.

In some embodiments, the method comprises increasing the rate of cardiac contractility modulation stimulations when measuring a rise in respiration rate, and decreasing the rate of cardiac contractility modulation stimulations when measuring decrease in respiration rate.

In some embodiments, the cardiac device is implanted in a patient diagnosed with heart failure.

In some embodiments, automatically setting one or more parameters of cardiac contractility modulation stimulation comprises selecting one or more intervals of the respiratory cycle for applying of cardiac contractility modulation stimulations.

In some embodiments, selecting comprises testing during which time interval(s) the applying does not cause or only least causes sensation to the patient.

In some embodiments, the method comprises selecting a time interval during the inhalation period for applying of cardiac contractility modulation stimulation.

In some embodiments, the method comprises selecting a time interval during the exhalation period for applying of cardiac contractility modulation stimulation.

In some embodiments, the method comprises recording an ECG of the patient and selecting a time for applying of cardiac contractility modulation stimulation when an amplitude of the R wave of a current cardiac cycle is higher than an average R wave amplitude calculated based on at least 5 previous cardiac cycles.

In some embodiments, the method comprises recording an ECG of the patient and selecting a time for applying of cardiac contractility modulation stimulation when an amplitude of the R wave of a current cardiac cycle is lower than an average R wave amplitude calculated based on at least 5 previous cardiac cycles.

In some embodiments, the method comprises recording an ECG of the patient and selecting a time for applying of cardiac contractility modulation stimulation when the RR interval of a current cardiac cycle is longer than an average RR interval calculated based on at least 5 previous cardiac cycles.

In some embodiments, the method comprises recording an ECG of the patient and selecting a time for applying of cardiac contractility modulation stimulation when the RR interval of a current cardiac cycle is shorter than an average RR interval calculated based on at least 5 previous cardiac cycles.

In some embodiments, measuring at least one respiration parameter comprises tracking the respiratory cycle to detect a dyspnea episode, and apply cardiac contractility modulation stimulation in response.

In some embodiments, the method further comprises assessing, according to the at least one measured respiration parameter, cardiac output and/or respiratory output over time and timing the applying of cardiac contractility modulation stimulation accordingly.

In some embodiments, the method comprises assessing if the patient has sensed the cardiac contractility modulation stimulation during a selected time interval of the respiratory cycle, and selecting a different time interval of the respiratory cycle for applying of the cardiac contractility modulation stimulation if the stimulation was sensed.

According to an aspect of some embodiments there is provided a system comprising:
an implantable cardiac device comprising:
at least one lead comprising one or more electrodes for applying cardiac contractility modulation stimulation to the heart; and
circuitry for controlling and activating the leads, the circuitry programmed to set at least one of timing and an intensity of the cardiac contractility modulation stimulation current according to a detected respiratory rate.

In some embodiments, the system comprises a sensor configured for detecting the respiratory rate.

In some embodiments, the sensor is selected from the group of: an acoustic sensor, a pulse oximeter, a pneumograph, a capnograph.

In some embodiments, the system comprises an intra-cardiac electrode for recording ECG; wherein the respiratory rate is determined according to the recorded ECG.

In some embodiments, the circuitry comprises a controller programmed to increase a rate of cardiac contractility modulation stimulations upon a detected rise in the respiratory rate and to decrease the rate of cardiac contractility modulation stimulations upon a decrease in the respiratory rate.

In some embodiments, the device comprises at least one lead comprising one or more electrodes for applying an electrical stimulation to the diaphragm and/or to the phrenic nerve.

In some embodiments, the circuitry comprises a controller programmed to control parameters of the stimulation applied to the diaphragm and/or phrenic nerves, the parameters comprising timing for applying the stimulation and an intensity of the stimulation current.

According to an aspect of some embodiments there is provided method for timing delivery of cardiac contractility modulation stimulation to the heart, comprising: determining if a cardiac contractility modulation stimulation applied to the heart caused pain and/or sensation to the patient;
assessing if the pain and/or sensation were associated with stimulation of the diaphragm, Phrenic nerve and/or its branches; and
setting one or more parameters for applying additional cardiac contractility modulation stimulation in accordance with the respiration cycle of the patient.

In some embodiments, setting one or more parameters comprises setting timing for applying of the additional cardiac contractility modulation stimulation, the timing including a time interval of the respiration cycle during which applying of the cardiac contractility modulation stimulation caused least pain or sensation to the patient.

In some embodiments, the cardiac contractility modulation stimulation is applied during a refractory period of the cardiac cycle.

According to an aspect of some embodiments there is provided a system comprising: one or more sensors for detecting parameters of a respiratory cycle in a patient; and an implantable cardiac device comprising:
at least one lead comprising one or more electrodes for applying cardiac contractility modulation stimulation to the heart; and
circuitry for controlling and activating the leads, the circuitry programmed to set parameters of the cardiac contractility modulation stimulation according to the parameters of the respiratory cycle detected by the one or more sensors.

In some embodiments, the one or more sensors are configured to detect parameters selected from the group of: respiration rate, relative timing of inhalation and exhalation, ECG of the heart.

In some embodiments, the parameters of the cardiac contractility modulation stimulation include timing of the cardiac contractility modulation stimulation, an intensity of the cardiac contractility modulation stimulation current.

In some embodiments, the circuitry is configured to set the parameters of cardiac contractility modulation stimulation in accordance with a relative anatomical space between the heart and the diaphragm and/or a relative anatomical space between the heart and the phrenic nerve, as correlated with the respiration cycle.

In some embodiments, the circuitry is configured to synchronize timing for applying the cardiac contractility modulation stimulation with timing in which the diaphragm is farthest from the heart and/or with timing in which the phrenic nerve is farthest from the heart.

In some embodiments, the circuitry is configured to time the applying the cardiac contractility modulation stimulation during an absolute refractory period of the cardiac cycle.

In some embodiments, the one or more sensors are selected from the group of: an acoustic sensor, a pulse oximeter, a pneumograph, a capnograph.

In some embodiments, the one or more sensors include an intra-cardiac electrode which records ECG, and wherein the determining at least one respiration parameter is based on the ECG recording.

In some embodiments, the circuitry is configured to set a current intensity of the of the cardiac contractility modulation stimulation to the highest level which does not cause pain.

In some embodiments, the circuitry is configured to set a rate for applying multiple cardiac contractility modulation stimulations.

In some embodiments, the circuitry is configured to increase the rate of cardiac contractility modulation stimulations when measuring a rise in respiration rate, and decrease the rate of cardiac contractility modulation stimulations when measuring decrease in respiration rate.

In some embodiments, the cardiac device is implanted in a patient diagnosed with heart failure.

In some embodiments, the circuitry is configured for testing, using the one or more sensors, during which time interval(s) applying of the cardiac contractility modulation stimulation does not cause or only least causes sensation to the patient.

In some embodiments, the circuitry is configured to time the applying the cardiac contractility modulation stimulation during an inhalation period.

In some embodiments, the circuitry is configured to time the applying the cardiac contractility modulation stimulation during an exhalation period.

In some embodiments, the circuitry is configured to time the applying the cardiac contractility modulation stimulation according to ECG recording obtained by the one or more sensors.

According to an aspect of some embodiments there is provided a method for timing delivery of cardiac contractility modulation stimulations to the heart via an implanted cardiac device, comprising:
tracking the respiration cycle in a patient;
identifying one or more time periods of the respiration cycle during which the diaphragm is non or least excitatory and/or non or least contractible; and
commanding the applying of cardiac contractility modulation stimulation during the identified one or more time periods.

In some embodiments, commanding is during a refractory period of the cardiac cycle.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1A is a flowchart of a method of setting at least one parameter of a cardiac contractility modulation stimulation to the heart in accordance with at least one respiration related condition, according to some embodiments;
FIG. 1B is a flowchart of a method for reducing or avoiding pain to the patient during cardiac contractility modulation stimulation, according tom some embodiments;
FIG. 1C is a schematic diagram for demonstrating factors taken into account when setting cardiac contractility modulation parameters, according to some embodiments;
FIG. 1D schematically illustrates a "by-activation" effect potentially caused by a cardiac contractility modulation signal applied to the heart as correlated with the distance from the heart, in accordance with some embodiments;
FIGs. 2A-B schematically illustrate a relative position of the heart, diaphragm and phrenic nerve during normal inhalation and exhalation, according to some embodiments;
FIG. 2C schematically illustrates anatomical changes in the heart in a patient, for example a heart failure patient;
FIG. 3A schematically illustrates an implanted device configured for applying cardiac contractility modulation stimulation, according to some embodiments;
FIG. 3B schematically illustrates a cardiac device comprising a plurality of leads, according to some embodiments;
FIG. 4 is an exemplary graph showing a relationship between the respiration cycle and the cardiac cycle (as recorded by ECG), in accordance with some embodiments;
FIG. 5 is a flowchart of a method for selecting one or more time intervals of the respiratory cycle for applying of cardiac contractility modulation stimulation, according to some embodiments;
FIG. 6 is a graph showing exemplary timing for applying of cardiac contractility modulation stimulation in correlation with the respiratory cycle, according to some embodiments;
FIG. 7 is a flowchart of a method for applying cardiac contractility modulation stimulation to the heart and for stimulating the diaphragm and/or phrenic nerve, according to some embodiments;
FIG. 8 is a block diagram of an implantable system configured for applying cardiac contractility modulation stimulation to the heart and for stimulating the diaphragm, according to some embodiments; and
FIG. 9 is a flowchart of a method for timing cardiac stimulation based on an excitatory state and/or contraction level of the diaphragm, according to some embodiments.

### DESCRIPTION OF SPECIFIC EMBODIMENTS OF THE INVENTION

The present invention, in some embodiments thereof, relates to cardiac contractility modulation (cardiac contractility modulation) treatment, and, more particularly, but not exclusively, to applying of cardiac contractility modulation stimulation in association with a respiratory stage or condition.

A broad aspect of some embodiments relates to cardiac contractility modulation stimulation which parameters are selected to reduce or prevent pain and/or sensation to the patient. Some embodiments relate to cardiac contractility modulation treatment which takes into account a patient's respiratory cycle when determining treatment parameters such as timing, stimulation current intensity, stimulation rate, treatment duration and/or other treatment parameters.

An aspect of some embodiments relates to timing of cardiac contractility modulation stimulation according to the respiratory cycle. In some embodiments, one or more time intervals and/or phases of the respiratory cycle are selected for applying cardiac contractility modulation stimulation. In some embodiments, the time intervals are selected as times in which a cardiac contractility modulation stimulation applied to the heart will not affect (or only least affect) the diaphragm or the phrenic nerve, such as by "side effect" stimulation generated by the cardiac contractility modulation signal. In some embodiments, the time intervals are selected based on a relative anatomical distance between the heart and the diaphragm and/or a relative anatomical distance between the heart and the phrenic nerve (and/or its branches). Since the relative anatomical distance varies for different stages of the respiratory cycle (such as at initiation of inhalation, peak of inhalation, initiation of exhalation, peak of exhalation, etc.)- applying cardiac contractility modulation at times in which the stimulated heart is at a larger distance from the diaphragm and/or its innervating nerves may reduce or prevent pain caused by by-stimulation of these tissues. Optionally, time intervals for applying cardiac contractility modulation are selected as times in which the diaphragm is farthest from the heart, for example at the peak of inhalation, so that stimulation applied to the heart will not affect (or only least affect) the diaphragm. In some embodiments, movement of the heart during the respiratory cycle and/or during the cardiac cycle is taken into account when setting parameters of the cardiac contractility modulation stimulation. In some embodiments, hemodynamic conditions and/or changes are taken into account when setting parameters of the cardiac contractility modulation stimulation. For example, the cardiac contractility modulation stimulation may be timed for when blood is due to flow into the heart, thereby potentially increasing the blood flow volume and/or rate.

In some embodiments, the cardiac contractility modulation stimulation rate and/or stimulation current intensity are controlled (e.g. by a controller of the implanted cardiac device) in accordance with the respiratory cycle. For example, the cardiac contractility modulation stimulation rate is increased if the respiration rate rises. For example, the cardiac contractility modulation stimulation rate is reduced if the respiration rate decreases. In some embodiments, the controller commands the electrifying of one or more leads of the cardiac device with the stimulation signal. Optionally, the command sets timing and/or intensity of the stimulation signal to be applied to the heart. In some embodiments, upon a command generated by the controller, electrical current is conducted via the one or more leads and optionally to tissue contacted by the one or more leads.

In some embodiments, characteristics of the respiratory cycle are tracked and/or estimated. In some embodiments, one or more sensors provide indications based on which the respiratory cycle is assessed. In some embodiments, the sensors are configured for measuring the respiratory cycle externally to the body. Such sensors may include, for example, an acoustic sensor (e.g. transducer); a pulse oximeter, a capnograph, a wearable device such as a pneumograph, an impedance sensor, and/or the like. Additionally or alternatively, one or more internal (e.g. implanted) sensors are used. In an example, an intra-cardiac electrode, an epicardial electrode and/or a subcutaneous electrode is configured for obtaining an ECG signal, and the respiratory cycle is calculated and/or estimated, for example according to a known correlation between the ECG signal and the respiratory cycle. In some embodiments, cardiac contractility modulation treatment parameters are determined according to measures of the ECG such as an amplitude of the QRS complex and/or an RR interval. For example, for tracking the respiratory cycle, the R wave amplitude and/or the RR intervals of the ECG signal may be tracked. A low R wave amplitude and a long RR interval may indicate the beginning of exhalation; a high R wave amplitude and a short RR interval may indicate the end of exhalation. In some embodiments, cardiac contractility modulation stimulation is timed to be delivered during a low R wave amplitude, for potentially enhancing contractility. Additionally or alternatively, cardiac contractility modulation stimulation is timed to be delivered during a high R wave amplitude to maximize contractility at its peak.

In some embodiments, pacing of the heart is provided, for example via one or more device electrodes. Optionally, signals for pacing the heart are delivered to synchronize the applying of cardiac contractility modulation (which is provided, in some embodiments, in the refractory period) with the respiration cycle. For example, the heart can be intentionally overpaced or under-paced, such as to deliver the cardiac contractility modulation stimulation in a selected time interval of the respiratory cycle.

In some embodiments, the time interval of the respiratory cycle suitable for delivering cardiac contractility modulation can be intentionally shifted, for example by pacing the heart and/or by stimulating (in an example, pacing) the diaphragm, for example as described hereinbelow.

An aspect of some embodiments relates to dual stimulation of the heart and the diaphragm (and/or its innervating nerves). In some embodiments, diaphragm contraction and/or relaxation are intentionally induced or enhanced by stimulating the diaphragm and/or the phrenic nerve (or its branches). In some embodiments, diaphragm stimulation is synchronized with cardiac contractility modulation stimulation. For example, stimulation may be applied to contract (or enhance contraction) of the diaphragm, and immediately thereafter cardiac contractility modulation stimulation may be applied to the heart. As the diaphragm, when contracted, may be farther from the heart as compared to a relaxed diaphragm position, applying cardiac contractility modulation at and/or immediately after the contraction may reduce or prevent pain or sensation to the patient.

A potential advantage of dual stimulation of the heart and the diaphragm may include improving, optionally simultaneously, cardiac contractility and breathing capacity and/or breathing rate. In some cases, a synergistic effect is produced due to one of these measures indirectly affecting the other (e.g. enhanced cardiac contractility may indirectly improve the breathing efficiency, for example as evident in clinical trials showing a higher peak oxygen uptake (peak VO2) in patients treated by cardiac contractility modulation). (See for example "Does Contractility Modulation Have a Role in the Treatment of Heart Failure?", Daniel Burkhoff, Curr Heart Fail Rep, DOI 10.1007/s11897-011-0067-3). In some embodiments, this synergistic effect may potentially reduce the required dosage and/or required total duration of cardiac contractility modulation treatment. In an example, dual stimulation may be advantageous for heart failure patients, who, in addition to malfunctioning of the heart, may suffer from respiratory muscle weakness which may lead to reduced breathing capacity.

In some embodiments, a system is provided, including an implantable pulse -generating device, one or more leads for applying cardiac contractility modulation stimulation to heart, and one or more leads for applying stimulation to the diaphragm and/or its innervating nerves. In some embodiments, the system comprises one or more sensors for tracking respiration, and cardiac contractility modulation treatment parameters and/or diaphragm stimulation parameters may be modified according to the indications received from these sensors. In some embodiments, the system comprises an ECG sensing electrode implanted in the heart, and cardiac contractility modulation treatment parameters and/or diaphragm stimulation parameters may be modified according to a recorded ECG signal. In some embodiments, pacing of the diaphragm is performed. By pacing the diaphragm, the respiratory cycle is at least partially controlled, potentially allowing for accurate synchronization of the cardiac contractility modulation stimulation relative to the respiratory cycle.

An aspect of some embodiments relates to timing cardiac stimulation according to an excitatory state and/or contraction state of the diaphragm. In some embodiments, cardiac stimulation such as cardiac contractility modulation stimulation is applied during a time period in which the diaphragm cannot or only least contract, for example, immediately following the peak of contraction. In some embodiments, cardiac contractility modulation stimulation is applied during a time period in which the diaphragm is non-excitatory or only least affected by stimulation. In some embodiments, the timing is selected for when there is an overlap between a non-refractory period of the cardiac cycle, and a non-excitatory/non contactable time period of the diaphragm. A potential advantage of timing cardiac contractility modulation stimulations according to an excitatory state and/or contracted state of the diaphragm may include reducing or preventing pain caused by undesired by-stimulation of the diaphragm and/or due to overcontracting of the diaphragm due to the by-stimulation.

As referred to herein, "timing" of a stimulation signal (e.g. cardiac contractility modulation signal) may refer to one or more of: timing relative to a measured and/or estimated cardiac cycle; timing relative to a measured and/or estimated respiratory cycle; timing relative to the refractory period of the heart (for example, when exactly during the refractory period to deliver the signal); timing relative to a sensed parameter, for example relative to a sensed contraction signal of the heart; timing relative to a measured and/or estimated anatomical distance; deciding if to deliver the signal within a certain cycle (the respiratory cycle or the cardiac cycle) or whether to skip one or more cycles and only then deliver the signal; defining a duration of the signal (pulse length); a pre-set timing (e.g. which was initially programmed in the device); and/or other settings of a relative or absolute times for delivery of the stimulation signal.

In some cases, timing of the stimulation signal and/or stimulation current intensity are selected and/or optimized per a specific patient. Optionally, inter-patient anatomical variability and/or breathing patterns affect the level of pain sensed by the patient.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

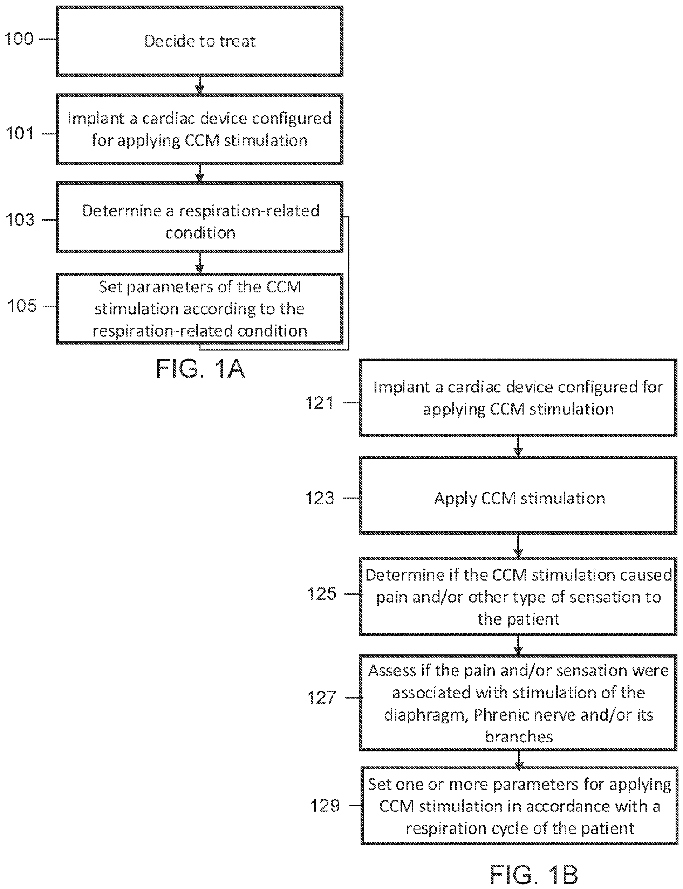

Referring now to the drawings, FIG. 1A is a flowchart of a method of setting at least one parameter of a cardiac contractility modulation stimulation to the heart in accordance with at least one respiration related condition, according to some embodiments.

In some embodiments, a decision is made to treat a patient by applying of cardiac contractility modulation stimulation to the heart (100). In some embodiments, the cardiac contractility modulation signal is a non-excitatory signal applied to the heart, optionally during a relative and/or absolute refractory period of the heart. In some embodiments of the invention, the signal is selected to increase the contractility of a cardiac ventricle when the electric field of the signal stimulates such ventricular tissue, for example, the left ventricle, the right ventricle and/or a ventricular septum. In some embodiments of the invention, contractility modulation is provided by phosphorylation of phospholamban caused by the signal. In some embodiments of the invention, contractility modulation is caused by a change in protein transcription and/or mRNA creation caused by the signal, optionally in the form of reversal of a fetal gene program.

The term "cardiac contractility modulation" is used herein, unless otherwise noted, as a general placeholder for all such signals. It is noted that in some embodiments the cardiac contractility modulation signal may be excitatory to tissue other than that to which it is applied. Various mechanisms by which cardiac contractility modulation signals may operate are described, for example in "Cardiac contractility modulation: mechanisms of action in heart failure with reduced ejection fraction and beyond" by C. Tschope et al, European Journal of Heart Failure (2018), doi:10.1002/ejhf.1349 and may serve to guide in selecting signal application parameters in order to utilize and/or comply with one or more of these mechanisms.

In some embodiments, a patient selected for treatment is a patient suffering from heart failure, congestive heart failure, and/or like symptoms. In some embodiments, a patient selected for treatment is a patient in which the heart pumping action is impaired, potentially affecting blood flow and/or oxygen supply. In some embodiments, a patient selected for treatment is a patient whose cardiac output and/or cardiac contractility are impaired, and may be improved by applying of cardiac contractility modulation therapy. In some cases, one or more effects of the cardiac contractility modulation treatment, such as a higher peak oxygen uptake, may improve breathing.

In some embodiments, a cardiac device configured for applying cardiac contractility modulation stimulation is implanted in the patient (101). In some embodiments, the device comprises a pulse generator which is optionally implanted outside the heart, for example in the subclavian area, and one or more leads for stimulating the heart, optionally contacting the ventricular septum.

In some embodiments, a respiration related condition is determined (103). In some embodiments, the condition is assessed per a specific patient. The respiration related condition comprises, in some embodiments, parameters of the respiration cycle, for example: a respiratory rate, a relative timing of exhalation and/or inhalation, a duration of inhalation and/or a duration of exhalation, and/or other. The respiration related condition comprises, in some embodiments, an anatomical parameter, for example: a relative anatomical space between the heart and the lungs, an anatomical space between the heart and the phrenic nerve, an anatomical space between the heart and the diaphragm, a distance between the stimulating lead(s) and anatomical structures, such as a distance between the stimulating lead(s) and the phrenic nerve (right phrenic nerve and/or left phrenic nerve).

In some embodiments, the respiration related condition is estimated and/or measured using one or more sensors. In some embodiments, parameters of the respiration cycle are determined according to input from one or more sensors.

In some embodiments, one or more implantable sensors are used, optionally implanted along with the cardiac contractility modulation stimulation device. In an example, an intra-cardiac electrode measures and optionally records an electrocardiogram. Optionally, according to the R-R interval, the heart rate is calculated.

In some embodiments, the intra-cardiac electrode used for ECG measurement is the same electrode which applies the cardiac contractility modulation stimulation.

In some embodiments, one or more external non-invasive sensors are used for measuring and/or estimating the respiration related condition. For example, an acoustic sensor (e.g. transducer and/or other sensor suitable for measuring sounds indicative of respiration); a pulse oximeter, a capnograph, a wearable device such as a pneumograph, impedance sensor. In some embodiments, respiration is measured and/or monitored by tracking (e.g. counting) rises of the chest.

In some embodiments, the one or more sensors are in communication with the implanted cardiac device, for example transferring signals to the device controller.

In some embodiments, respiratory parameters (e.g. rate) are monitored continuously. Additionally or alternatively, periodic measurements are performed, for example when configuring the cardiac device before, during, and/or following implantation.

In some embodiments, parameters of the cardiac contractility modulation stimulation are set (e.g. selected and programmed into the cardiac device controller) according to the determined respiration related condition (105). Parameters of the cardiac contractility modulation treatment include, for example: dosage (e.g. timing of stimulation, number of stimulations); stimulation current intensity; safety thresholds; stimulation rates, and/or other parameters.

**FIG. 1B** is a flowchart of a method for reducing or avoiding pain to the patient during cardiac contractility modulation stimulation, according tom some embodiments.

In some embodiments, stimulation applied by the implanted cardiac device may cause sensation or pain to the patient. Such sensation may be a result of direct and/or indirect stimulation of nerves by the cardiac device, such as nerves located along a stimulation path through which electrical current is passed. In some cases, the device stimulation path passes near and/or through innervated tissue, potentially causing discomfort or even pain to the patient.

In some cases, stimulation applied by the cardiac device affects innervated tissue of the diaphragm and/or its innervating nerves, such as the Phrenic nerve and/or its branches (e.g. left and/or right Phrenic nerves).

In some embodiments, reducing or avoiding sensation the patient may be achieved by one or more of: reducing the intensity of the stimulation current, reducing the number of stimulations, reducing or stopping stimulation in the event pain is felt. However, these modifications may result in sub-optimal cardiac contractility modulation treatment.

The following method, in accordance with some embodiments, describes applying of cardiac contractility modulation which takes into account reduction or avoidance of sensation and/or pain to the patient, which, in some cases, may be associated with Phrenic nerve activation and/or diaphragm activation. In some embodiments, reduction or avoidance of pain associated with phrenic nerve activation are achieved by synchronizing the cardiac contractility modulation treatment with respiration parameters and/or conditions.

In some embodiments, a cardiac device configured for applying cardiac contractility modulation stimulation is implanted (121). In some embodiments, one or more cardiac contractility modulation stimulations are applied (123). In some embodiments, during and/or following applying of the cardiac contractility modulation stimulation, a determination is made as to whether the cardiac contractility modulation stimulation caused sensation or pain (125), for example by asking the patient to report what they felt.

In some embodiments, if sensation or pain were caused by the stimulation, assessment of the source of pain is performed, for example, whether the sensation or pain were caused by activation and/or stimulation of the Phrenic nerve or its branches (127). Optionally, the assessment is based on a reporting from the patient, indicating the pain level and/or the body area in which the pain was sensed.

In some embodiments, one or more parameters for applying cardiac contractility modulation stimulation are set in accordance with a respiration cycle of the patient (129). As the Phrenic nerve (and/or its branches) are expected to move with breathing, for example, be pushed by the lungs towards the heart during inhalation; be pushed towards the heart by the diaphragm during exhalation; and/or other movements or relative changes in position, setting the cardiac contractility modulation stimulation in synchronization with the breathing cycle may reduce or prevent pain, such as by reducing the stimulating effects of the cardiac contractility modulation signal on the Phrenic nerve or its branches. In some embodiments, reduction of pain or sensation is achieved by delivering the cardiac contractility modulation signal at times when the phrenic nerve is farther from the heart.

In some embodiments, parameters of the cardiac contractility modulation treatment are set with respect to the respiration cycle. In some embodiments, parameters of the cardiac contractility modulation treatment are set with respect to the cardiac cycle.

In some embodiments, parameters of the cardiac contractility modulation treatment are set upon first and/or periodic configuring of the cardiac device, such as in a lab or clinic. In some embodiments, settings are selected based on the patient anatomy- for example, variations in anatomy between patients may cause sensation and/or pain to some patients while other patients may not sense or not be discomforted by the applied stimulation.

In some embodiments, cardiac contractility modulation parameters such as timing of the signal are selected following implantation, optionally immediately following implantation. In some embodiments, the timing is selected in accordance with a respiration rate, optionally measured for the specific patient. In some embodiments, cardiac contractility modulation timing is matched in real time to a tracked respiration rate. In some embodiments, respiration is monitored continuously, for example based on the ECG recording and/or based on input received from a sensor, and closed loop control is performed for setting the cardiac contractility modulation parameters (e.g. timing and/or currently intensity). In an example, in a patient engaged in physical activity, a higher respiration rate may be detected, and the rate of applying cardiac contractility modulation stimulation may be raised in response. Optionally, the rate of cardiac contractility modulation is raised so as to deliver a stimulation within a refractory period of each cardiac cycle.

In some embodiments, an average respiration rate is deduced for a patient, and cardiac contractility modulation timing and/or stimulation current intensity are set in accordance with the average rate.

In some embodiments, applying of stimulations is set and/or controlled in response to changes in the respiration rate. For example, in some embodiments, if an increase in the respiration rate is sensed (e.g. by the one or more sensors), cardiac contractility modulation may be applied at a higher rate, optionally timed for selected interval(s) of the breathing cycle.

In some embodiments, parameters of cardiac contractility modulation are set and/or controlled by a user, e.g. a physician. Additionally or alternatively, parameters of cardiac contractility modulation treatment are set and/or controlled by a patient, for example, a patient may request to shorten or stop applying of a stimulation, and/or reduce stimulation intensity, if pain is sensed. Additionally or alternatively, parameters of cardiac contractility modulation treatment are set and/or controlled automatically, for example by the device controller. Optionally, parameters are controlled in response to indications received from one or more sensors, such as indications pertaining to a currently sensed respiration rate.

A potential advantage of setting cardiac contractility modulation parameters in relation to breathing may include utilizing natural anatomical changes which occur during breathing, such as the phrenic nerve or its branches being positioned farther from the heart during certain times of the respiration cycle, and/or the diaphragm moving farther from the heart during certain times of the respiration cycle, to potentially reduce pain to the patient caused by phrenic nerve activation and/or diaphragm activation.

In some embodiments, cardiac contractility modulation parameters are selected and/or controlled to improve breathing, optionally over time. For example, enhancing the heart's contraction ability may indirectly improve breathing. In some cases, such an in HF condition, patients suffer from low volume, "shallow" breathing. Improving the heart's contraction in such patients may improve the breathing capacity. In another example, if a dyspnea episode is detected in a patient in which the cardiac device is implanted, cardiac contractility modulation may be applied at a higher rate and/or strength to potentially improve breathing.

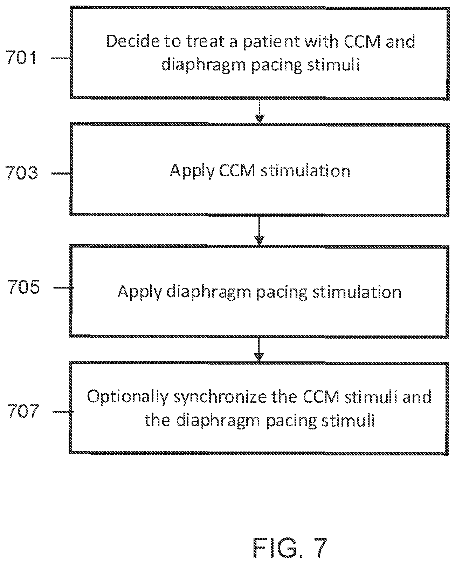

In some embodiments, cardiac contractility modulation stimulation is delivered to intentionally stimulate the diaphragm. Optionally, indirect stimulation occurs when cardiac contractility modulation stimulation is applied to the heart. Additionally or alternatively, in some embodiments, the implanted device is configured to stimulate the diaphragm directly by stimulating the diaphragm itself and/or the phrenic nerve, for example as further described in FIGs. 7-8 below.

In some embodiments, an accelerometer and/or motion sensor is provided, for detecting a posture or a change in posture. Optionally, parameters of the cardiac contractility modulation stimulation are selected based on input from the accelerometer, for example, the stimulation is timed for when a patient's posture is one in which least or no pain is sensed during applying of cardiac contractility modulation. Such posture (or a set of postures/ range of positions) may be detected during initial testing, and device settings may be configured accordingly.

In some embodiments, the implanted device may be configured for delivering signals that partially or fully block a nerve. Optionally, nerve-blocking signals are delivered right before applying of cardiac contractility modulation. In an example, conduction via the phrenic nerve may be partially blocked to reduce or avoid pain associated with activation of the diaphragm by the phrenic nerve.

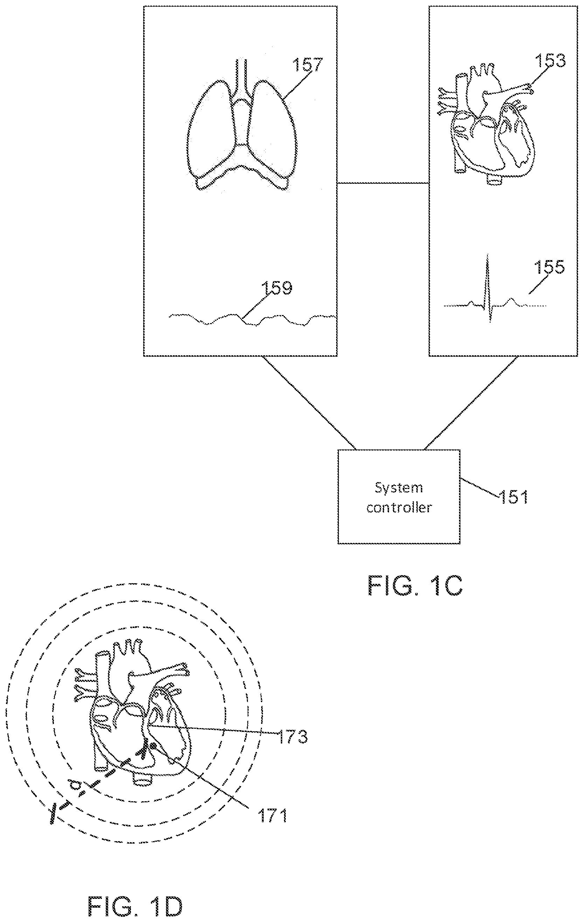

**FIG. 1C** is a schematic diagram for demonstrating factors taken into account when setting cardiac contractility modulation parameters, according to some embodiments.

In some embodiments, the system controller 151 is programmed with and/or is configured for automatic selection of cardiac contractility modulation settings, including, for example, timing for delivering the signal, an intensity of the signal, a rate for applying the signal, a duration of a treatment session (e.g. stimulations provided over several minutes and/or hours), a total treatment duration (e.g. overall treatment for a duration over weeks, months).

In some embodiments, cardiac contractility modulation parameters are selected taking into account one or more parameters related to the heart 153, for example: the cardiac cycle (for example as indicated by an ECG recording 155); a relative anatomical position of the heart (optionally per patient and/or per respiratory phase or condition); a contractility level of the heart; a size and/or mass of the heart; pumping ability; cardiac output; stroke volume, ejection fraction, HF etiology, and/or general patient parameters such as height, BMI, co-morbidities, sex, age.

In some embodiments, cardiac contractility modulation parameters are selected taking into account one or more parameters related to the respiratory system 157, for example: the respiratory cycle (for example as indicated by a recorded respiration signal 159); the respiration rate; anatomical positions and changes thereof of during the respiration cycle (e.g. of the lungs, diaphragm, phrenic nerves, rib cage, etc.); respiratory phases and their length; lung capacitance; respiration depth.

In some embodiments, cardiac contractility modulation parameters are selected taking into account hemodynamic effects, for example, flow of blood to and/or from the heart, optionally in association with a respiratory phase (e.g. inhalation, exhalation, peak of inhalation, end of exhalation, and/or other).

In some embodiments, cardiac contractility modulation is timed to be delivered during a specific respiratory phase, for example during inhalation or during exhalation. In such situation, the cardiac contractility modulation may not be delivered at each cardiac cycle, but for example at an alternating manner (one cardiac cycle -yes, the next cardiac cycle- not), and/or in other patterns. In some embodiments, to compensate for "missed" cardiac cycles (i.e. a cycle in which cardiac contractility modulation was not delivered), treatment parameters such as the duration of treatment, the current intensity, the number of cardiac contractility modulation stimulations delivered during a cardiac cycle and/or during a respiratory cycle, and/or other parameters are modified. For example, the treatment duration is lengthened. For example, the stimulation current amplitude is reduced to allow a stimulation that is longer in duration. For example, the stimulation current amplitude is increased to strengthen contraction.

**FIG. 1D** schematically illustrates a "by-activation" effect potentially caused by a cardiac contractility modulation signal applied to the heart as correlated with the distance from the heart, in accordance with some embodiments.

In some embodiments, cardiac contractility modulation stimulation is applied to the heart, for example by an electrode 171 positioned in contact with the ventricular septum 173. In some embodiments, the applied electrical signal may cause (in addition to intentional enhancing of contractility) "by-activation" of nerve tissue and/or nearby organs. For example, the cardiac contractility modulation signal may stimulate the left and/or right phrenic nerves passing adjacent the heart, the diaphragm.

In some cases, "by activation" of nerve tissue and/or nearby organs may result in pain or sensation to the patient. In some embodiments, to reduce or prevent pain, a "by-activation" distance (indicated as "d" in the figure) may be calculated or estimated. Optionally, by timing the cardiac contractility modulation stimulation to when the by-activated nerves and/or by-activated organs are as far as possible from the heart, pain may be reduced or prevented.

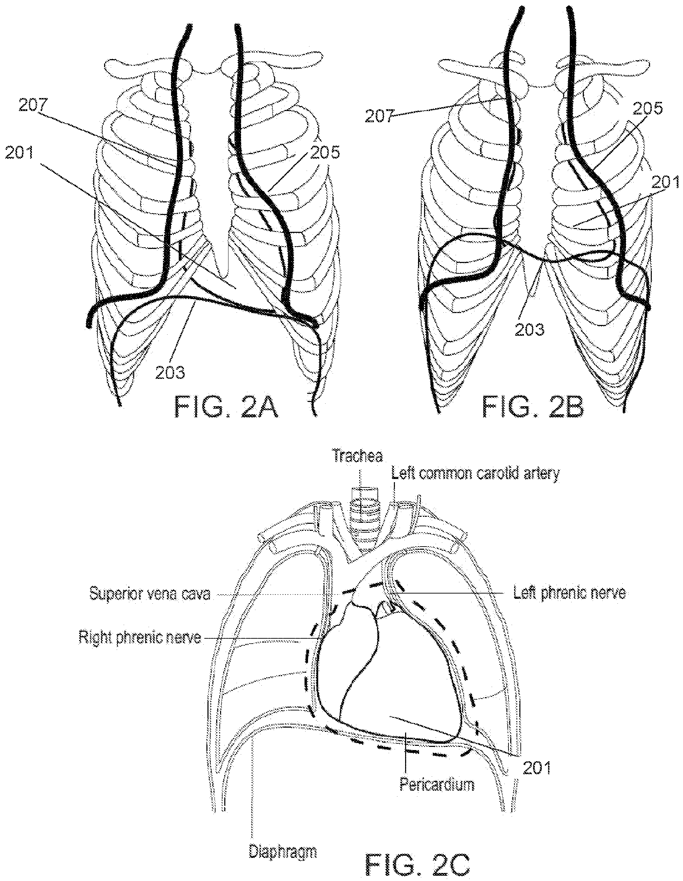

**FIGs. 2A-B** schematically illustrate a relative position of the heart, diaphragm and phrenic nerves during normal inhalation and exhalation, according to some embodiments.

FIG. 2A illustrates a relative position of the heart 201, diaphragm 203, and phrenic nerves (left 205 and right 207) at the peak of inhalation. As can be observed, during inhalation, the diaphragm contracts and drawn downwards, creating a vacuum in the thoracic cavity which provides for inflation of the lungs.

FIG. 2B illustrates a relative position of the heart 201, diaphragm 203, and phrenic nerves (left 205 and right 207) at the end of exhalation. As can be observed, during exhalation, the diaphragm relaxes and is moved into closer proximity with the heart, allowing the lungs to deflate.

In some embodiments, cardiac contractility modulation treatment parameters are set based on the relative anatomical positions of the heart and diaphragm and/or the heart and the phrenic nerve, during breathing. For example, in some embodiments, applying of the cardiac contractility modulation signal is set to when the diaphragm is farthest from the heart, e.g. during or at the peak of inhalation. For example, applying of the cardiac contractility modulation signal is set to when the phrenic nerve (e.g. right and/or left branches) are farthest from the heart.

Other distances and/or relative anatomical positions may be taken into account when timing the cardiac contractility modulation signal, for example, expansion and contraction of the rib cage, lung inflation and deflation, contraction of the heart, chest muscle movement and/or other.

FIG. 2C schematically illustrates anatomical changes in the heart in a patient, for example in a heart failure patient. In some cases, in a patient suffering from a cardiac condition such as HF, the heart 201 may be enlarged in size, such as due to a constant demand to pump more blood. In some cases, the heart may develop more muscle mass. The enlarged heart is indicated in this figure by the dashed line.

In some cases, the left ventricle is enlarged, optionally more than other portions of the heart. In some cases, anatomical changes in the heart size, dimension and/or position may cause the heart to be closer to the diaphragm and/or to the phrenic nerves passing by it. Therefore, in such cases, applying cardiac contractility modulation stimulation to the enlarged heart may be more prone to affecting (e.g. by neural stimulation of) nearby structures such as the diaphragm and/or phrenic nerves.

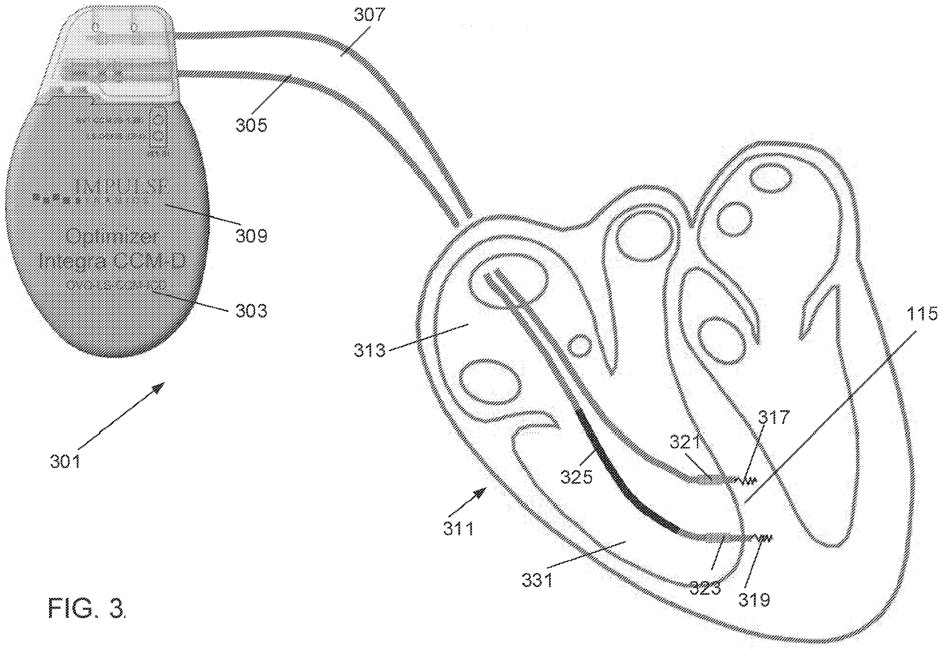

**FIG. 3A** schematically illustrates an implanted device configured for applying cardiac contractility modulation stimulation, according to some embodiments.

In some embodiments, implantable device 301 comprises a pulse generator 303. In some embodiments, pulse generator 303 comprises a housing 309 which encases, for example: powering means (e.g. a battery), control circuitry (e.g. a controller) configured for timing and generating the electrical pulses, sensing circuitry, communication circuitry, memory means, and/or other operational modules.

In some embodiments, one or more stimulation leads such as 305, 307 are connected to the housing and extend externally from it. In some embodiments, a lead comprises one or more electrical wires, surrounded by an external insulating layer. In some embodiments, a lead is comprised of two wires, having different polarities. In some embodiments, a wire of the lead is coiled.

In some embodiments, pulse generator 303 is implanted outside the heart, for example in the subclavian area. Optionally, implantation is via a minimally invasive procedure.

In some embodiments, a housing of pulse generator 303 is implanted subcutaneously, in proximity of the left chest.

In some embodiments, leads 305 and 307 extend from pulse generator 303, and at least a distal segment of the leads is implanted within the heart 311. In some embodiments, as shown, both leads are passed through the right atrium 313, and contact, at their distal ends, the ventricular septum 315. In some embodiments, each lead contacts the septum at a different location.

It is noted that additionally or alternatively, a single lead which includes two spaced apart stimulation electrodes may be used.

It is also noted that while the figures shows both leads being positioned in the right ventricle 331 against ventricular septum 315, one or more stimulating leads may be in other locations, with consequently different effect circles and/or targeting different tissues. In some embodiments, the leads are located inside the heart, on the right side thereof optionally to take advantage of two potential advantages: a. less out-of-heart tissue being stimulated; and b. less invasive access and/or presence than in the left heart.

In some embodiments, one of the leads is implanted outside the heart, and the other lead is implanted inside the heart.

In some embodiments, each of the leads ends with a tip electrode (see 317 of lead 307, 319 of lead 305). The tip electrode may be configured as a contact electrode, a screw-in electrode, a sutured electrode, a free-floating electrode and/or other types.

In some embodiments, one or both of the leads includes a ring electrode (see 321 of lead 307, 323 of lead 305), located along the lead, proximally to the tip electrode.

In some embodiments, the electrodes are implanted in the right ventricle or in the right atria of the heart.

In some embodiments, a tip electrode is formed with a threading so as to be threaded into the tissue. Alternatively, a tip electrode is solely placed in contact with the tissue.

In some embodiments, one or both of the leads includes a defibrillation coil (see 325 of lead 305). Optionally, coil 325 is located along the lead, proximally to the tip electrode and/or proximally to the ring electrode.

In some embodiments, the coil is implanted in the right ventricle, right atria or in the vena cava.

In some embodiments, one or both leads are configured to deliver a non-excitatory signal such as a cardiac contractility modulation signal.

In some embodiments, the cardiac contractility modulation signal is applied in contact with the ventricular tissue or within ventricular tissue.

In some embodiments, the cardiac contractility modulation signal is applied to the heart during a relative and/or absolute refractory period of the heart. In some embodiments, the signal is selected to increase the contractility of a cardiac ventricle when the electric field of the signal stimulates such ventricular tissue, for example, the left ventricle, the right ventricle and/or a ventricular septum. In some embodiments of the invention, contractility modulation is provided by phosphorylation of phospholamban caused by the signal. In some embodiments of the invention, contractility modulation is caused by a change in protein transcription and/or mRNA creation caused by the signal, optionally in the form of reversal of a fetal gene program. The term "cardiac contractility modulation" is used herein, unless otherwise noted, as a general placeholder for all such signals. It is noted that in some embodiments the cardiac contractility modulation signal may be excitatory to tissue other than that to which it is applied.

While not being limited to a single pulse sequence, the term cardiac contractility modulation is used to describe any of a family of signals which includes a significant component applied during an absolute refractory period and which has a clinically significant effect on cardiac contractility in an acute and/or chronic fashion and/or which causes a reversal of fetal gene programs and/or which increases phosphorylation of phospholamban. In some embodiments, the signal is potentially excitatory to one part of the heart but non-excitatory to other parts. For example, a signal can be excitatory in atria, but applied at timing (relative to ventricular activation) when it is not excitatory in the ventricle.

In some embodiments of the invention, the signal while potentially stimulatory during the receptive period of the cardiac cycle, is non-excitatory due to its timing. In particular, the signal is applied during the refractory period of the tissue which is affected by it and, optionally, within the absolute refractory period.

In some embodiment, a device electrode, for example the cardiac contractility modulation applying electrode, is used for measuring the R wave amplitude and/or RR interval of the cardiac cycle.

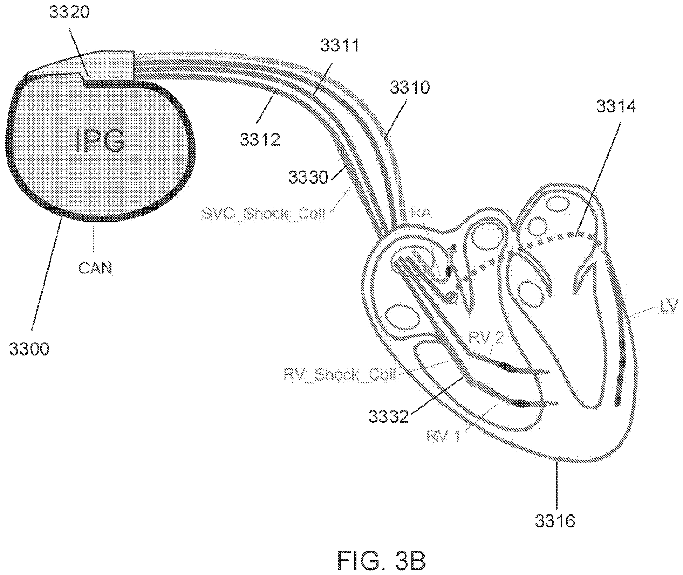

**FIG. 3B** schematically illustrates a cardiac device comprising a plurality of leads, according to some embodiments. In some embodiments, device 3300 is configured for delivering cardiac contractility modulation stimulations to the heart. In some embodiments, device 3300 is further configured to function as a cardiac defibrillator (ICD).

In some embodiments, device 3300 comprises a plurality of leads. In the example shown, a first lead 3310 extends to the right atrium of the heart 3316; a second lead 3311, such as for applying cardiac contractility modulation stimulation, extends to the right ventricle, optionally contacting the ventricular septum; a third lead 3312 extends to the right ventricle, optionally contacting the ventricular septum; and a fourth lead 3314 extending to the left ventricle, for example through the coronary sinus. In some embodiments, one or more of the leads comprises a defibrillation coil. In this example, lead 3312 comprises a superior-vena cava shock coil 3330 and a right ventricle shock coil 3332.

In some embodiments, the plurality of leads are connected to the device housing 3320 via a plurality of ports (not shown). In some embodiments, control of activation of one or more leads is via switch circuitry, for example switch circuitry of the device controller.

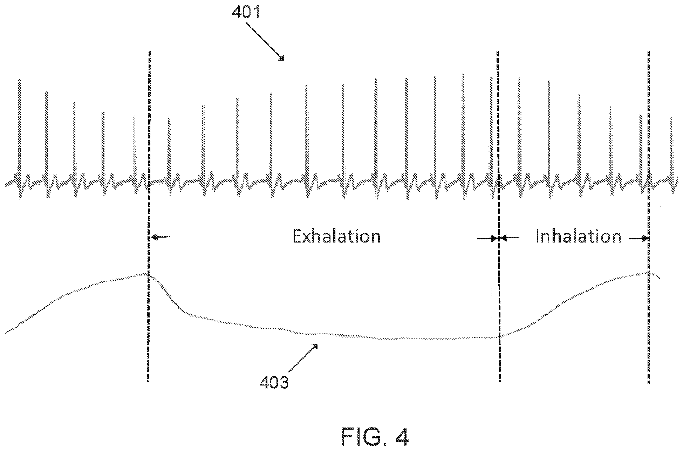

**FIG. 4** is an exemplary graph showing a relationship between the respiration cycle and the cardiac cycle (as recorded by ECG), in accordance with some embodiments.

The graph presents an ECG signal 401 (voltage vs. time) recorded in correlation to tracking of breathing 403. In this example, a thoracic impedance sensor was used for measuring breathing.

In some embodiments, for determining timing and/or rate for delivering cardiac contractility modulation signals, cardiac cycle characteristics are measured or assessed. In some embodiments, an ECG recording is obtained and measures of the signal are determined or calculated. For example, signal amplitudes are tracked, such as the R wave amplitude. For example, time intervals are tracked, such as the RR interval.

In some embodiments, R wave amplitude parameters including, for example, peak, average, minimum (RWP, RWA, RWM) are measured. In some embodiments, RR interval parameters such as longest interval, shortest interval, and average interval are measured. Optionally, measuring is over a plurality of cardiac cycles, for example 2, 10, 100, 1000 cardiac cycles or intermediate, higher or smaller number.

In some embodiments, for setting cardiac contractility modulation parameters, measurements obtained during a current cardiac cycle are compared to parameters obtained from one or more previous cardiac cycles. In some embodiments, cardiac contractility modulation stimulation is set to be delivered in cardiac cycles which parameters comply with a selected criteria, for example, cardiac cycles in which:
The R wave amplitude is higher than RWA;
The R wave amplitude is higher than RWA+ (RWP-RWA)/2;
The R wave amplitude is lower than RWA;
The R wave amplitude is lower than RWA-(RWA-RWM)/2;
The RR interval is longer than an average RR interval;
The RR interval is shorter than an average RR interval;
and/or other criteria.

In some embodiments, if a currently measured parameter of a cardiac cycle is within the criteria, the cardiac contractility modulation stimulation is delivered during the ventricle refractory period of that cardiac cycle.

As can be observed from the exemplary graph, the cardiac cycle is correlated to the respiration cycle. For example, the heart rate (as indicated by the reduction in RR intervals) increases at the end of exhalation, and decreases at the end of inhalation.

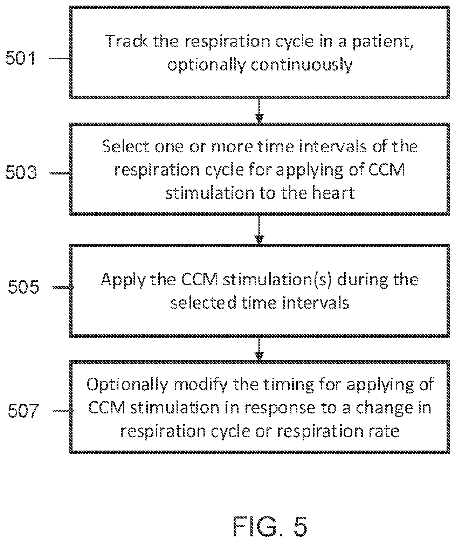

**FIG. 5** is a flowchart of a method for selecting one or more time intervals of the respiratory cycle for applying of cardiac contractility modulation stimulation, according to some embodiments.

In some embodiments, cardiac contractility modulation treatment parameters such as timing of the stimulation pulse, a length of the pulse, a rate of applying stimulation pulses, a current intensity of the pulse are selected in synchronization with the respiration cycle.

In some embodiments, the cardiac contractility modulation stimulation is applied during one or more selected time intervals of the respiration cycle.

In some embodiments, the respiration cycle of a patient is tracked, optionally continuously (501). Optionally, the respiration cycle is determined by measuring breathing directly, for example using one or more sensors as described hereinabove. Additionally or alternatively, the respiration cycle is deduced or calculated from other measures, such as from a recorded ECG signal.

In some embodiments, one or more time intervals of the respiration cycle are selected for applying of cardiac contractility modulation stimulation.

Some examples of time intervals may include:
* applying of the cardiac contractility modulation stimulation during a time interval which is shorter than the full respiration cycle (i.e. a full cycle including a single full inhalation and a single full exhalation); optionally, the time interval starts at the initiation of inhalation;
* applying of the cardiac contractility modulation stimulation during a time interval which is between 0-100% of the inhalation period;
* applying of the cardiac contractility modulation stimulation during a time interval which is between 0-100% of the exhalation period;
* applying of the cardiac contractility modulation stimulation following an RR interval which is shorter than a set percentile of RR intervals measured during one or more previous cardiac cycles (e.g. shorter than at least 70%, at least 80%, at least 90% of RR intervals measured in previous 2, 3, 4, 5, 6, 10, 20, 30, 100 or intermediate, larger or smaller number of cardiac cycles). In some embodiments, cardiac contractility modulation is delivered during a ventricle refractory period, within a time period of 20-100 milliseconds, 30-60 milliseconds, 50-120 milliseconds or intermediate, longer or shorter time periods following detection of the R wave.
* applying of the cardiac contractility modulation stimulation following an RR interval which is longer than a set percentile of RR intervals measured during one or more previous cardiac cycles; (e.g. longer than at least 70%, at least 80%, at least 90% of RR intervals measured in previous 2, 3, 4, 5, 6, 10, 20, 30, 100 or intermediate, larger or smaller number of cardiac cycles).

In some embodiments, the time interval(s) for applying the cardiac contractility modulation stimulation(s) is selected using optimization testing. Optionally, optimization testing is performed during or right after implantation, when configuring the device settings. During such optimization testing, the respiration cycle of a patient may be divided into multiple time periods, each time period being shorter than the full cycle (for example, a full cycle is divided into 2, 3, 4, 5, 6 or intermediate, smaller or larger number of time periods). In some embodiments, during each time period, a cardiac contractility modulation stimulation is applied. Optionally, the current intensity of the applied signal is gradually increased, until reaching an intensity which is sensed by the patient. Then, in some embodiments, a time period for delivering of the cardiac contractility modulation stimulation is selected as the time period during which the highest current intensity value that caused no pain or sensation was applied.

In some embodiments, the cardiac device is programmed to apply the cardiac contractility modulation stimulation(s) at the selected time intervals (505). Optionally, if the patient senses pain, the timing and/or intensity of the stimulation is modified. Optionally, a different time interval is selected for applying the signal.

Optionally, the timing for applying cardiac contractility modulation is modified in response to a change in the respiration cycle or respiration rate (507). For example, in some embodiments, the device is programmed to increase a rate of the cardiac contractility modulation stimulations when sensing a rise in the respiration rate; and/or decrease a rate of the cardiac contractility modulation stimulations when sensing a drop in the respiration rate. Optionally, monitoring of respiration is performed in real time, and indications of a current respiration rate and/or a change in the respiration rate are communicated to the device controller (such as from one or more sensors that track the respiration rate. In some embodiments, the device controller modifies the cardiac contractility modulation treatment (dosage, timing, etc.) based on closed loop feedback from the sensors. In an example, the device is configured to change parameters of the cardiac contractility modulation treatment in response to detection of respiratory distress, for example as may be evident from tracking the respiration rate. In an example, respiratory distress causes an elevated respiration rate; to potentially prevent or reduce "over activating" of the diaphragm (by unintentional stimulation of the diaphragm itself and/or of the phrenic nerves), cardiac contractility modulation may be delivered at the initiation of inhalation when the diaphragm and/or phrenic nerves may be far enough from the heart, and thereby far enough from the stimulating electrode(s).

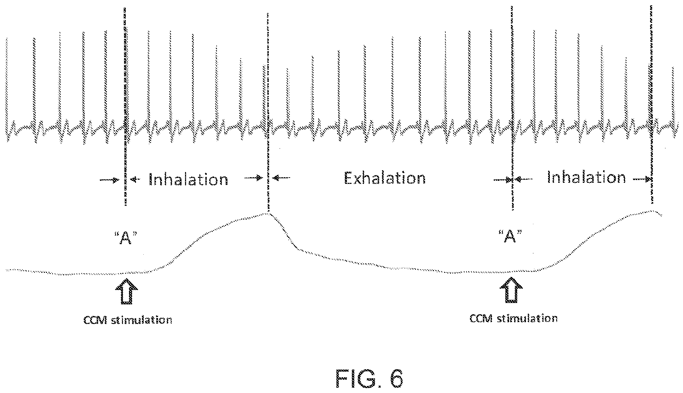

**FIG. 6** is a graph showing exemplary timing for applying of cardiac contractility modulation stimulation in correlation with the respiratory cycle, according to some embodiments.

In the example shown, cardiac contractility modulation stimulation is applied, for example, at the initiation of inhalation, indicated at point "A". In some embodiments, a duration of the applied cardiac contractility modulation pulse is between, for example, 20msec-30msec, 15msec-40msec, 30msec-45 msec, 25msec-50msec, or intermediate, longer or shorter duration.

**FIG. 7** is a flowchart of a method for applying cardiac contractility modulation stimulation to the heart and for stimulating the diaphragm and/or phrenic nerve, according to some embodiments.

In some embodiments, the diaphragm and/or its innervating nerves are intentionally stimulated. Optionally, stimulation is applied to strengthen contraction of the diaphragm. Optionally, stimulation is applied to induce relaxation of the diaphragm.

In some embodiments, a physician (and/or other medical personnel) decides to treat a patient with both cardiac contractility modulation and diaphragm pacing stimuli (701).

In some embodiments, a cardiac contractility modulation device is implanted in the patient. In some embodiments, the device includes, in addition to the cardiac contractility modulation leads, one or more leads configured for delivering stimuli to the diaphragm and/or to the phrenic nerve(s). In some embodiments, the diaphragm pacing leads extend from the implanted device housing to the diaphragm muscle, optionally contacting at upper opposing side locations.

In some embodiments, cardiac contractility modulation stimulation is applied (703), for example as described herein.

In some embodiments, diaphragm pacing stimulation is applied (705). The applied stimuli may cause the diaphragm to contact, inducing inhalation of the user. In some embodiments, stimulation is applied directly to the tissue of the diaphragm. Additionally or alternatively, stimulation is applied to the phrenic nerves.

In some embodiments, the cardiac contractility modulation stimuli and the diaphragm pacing stimuli are synchronized (707). Optionally, timing of the cardiac contractility modulation stimulation is set relative to the diaphragm pacing stimulation. For example, cardiac contractility modulation stimulation is applied immediately after applying the diaphragm pacing stimulation, when the diaphragm contracts and may be at its farthest position from the heart.

A potential advantage of delivering both cardiac contractility modulation stimulation and stimulation to the diaphragm may include improving, optionally simultaneously, the heart's ability to contract as well as the breathing capacity of the patient. Stimulating the heart to enhance its contraction in addition to stimulating the diaphragm to enhance breathing may lead to a synergistic effect, where the improved heart condition improves breathing and/or the improved breathing improves functioning of the heart. A dual treatment including heart and diaphragm stimuli may potentially reduce a total length of the treatment, improve a patient's wellbeing, reduce or prevent pain to the patient during applying of cardiac contractility modulation, and/or other advantages.

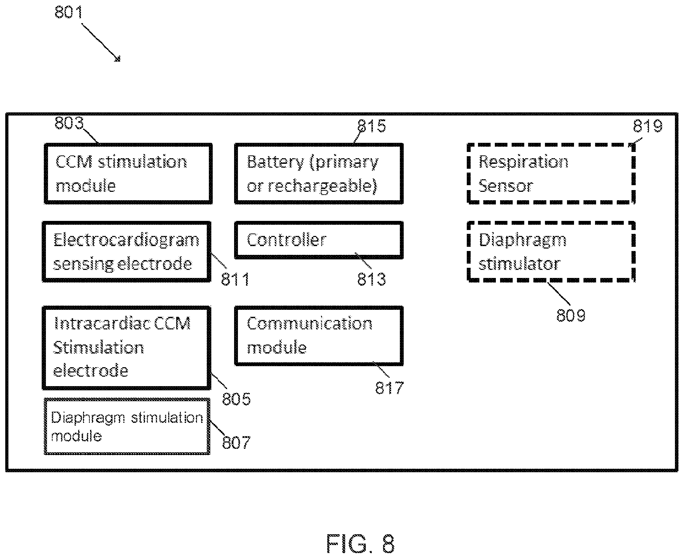

**FIG. 8** is a block diagram of an exemplary system configured for applying cardiac contractility modulation stimulation to the heart and for stimulating the diaphragm, according to some embodiments.

In some embodiments, a system 801 is configured for stimulating the heart and optionally for stimulating the diaphragm and/or its innervating nerves (e.g. phrenic nerve(s)). In some embodiments, the system (or components of the system) are implanted in a patient. In an example, a device housing includes circuitry configured for controlling and activating one or more leads which extend from the housing. In some embodiments, at least one lead enters the heart to stimulate it; and at least one lead extends to the diaphragm and/or extends to the phrenic nerve to stimulate them.

In some embodiments, one or more leads for stimulating the diaphragm are positioned through the right brachiocephalic vein and/or at the left pericardiophrenic vein, adjacent the phrenic nerves. Additionally or alternatively, one or more leads for stimulating the diaphragm are positioned through the azygos vein.

In some embodiments, system 801 comprises a controller 813. Optionally, the controller is programmed with operation settings such as: cardiac contractility modulation treatment dosage (e.g. timing, rate, stimulation strength), diaphragm pacing settings (e.g. timing, rate, stimulation strength), safety thresholds, and/or other control parameters.

In some embodiments, system 801 comprises a cardiac contractility modulation stimulation module 803, configured for activating at least one intra-cardiac cardiac contractility modulation stimulation electrode 805. In some embodiments, the system comprises a diaphragm stimulation module 807, configured for activating at least one diaphragm stimulator 809 (e.g. a lead including an electrode).

In some embodiments, the system comprises an ECG sensing electrode 811, optionally positioned inside the heart or externally to the heart. Optionally, sensing electrode 811 monitors the heart cycle continuously. Alternatively, sensing electrode 811 monitors the heart cycle periodically. Additionally or alternatively, sensing electrode monitors the heart cycle upon demand, for example in response to instructions received from controller 813.

In some embodiments, the system comprises a respiration sensor 819. The sensor may be an implantable sensor or an external sensor. The respiration sensor may monitor breathing continuously, periodically, and/or upon demand.

In some embodiments, the system comprises a power source, such as a battery 815. The battery may be a primary (e.g. replaceable) battery or a rechargeable battery.

In some embodiments, the system comprises a communication module 817. In some embodiments, the communication module sends and/or receives signals from one or more external devices. For example, the communication module receives input and/or sends data to a user interface. The user interface may be configured on a physician's computer, on a physician or patient cell phone app, on a lab based computer and/or other. In some embodiments, the communication module sends and/or receives data from a cloud server. Optionally, data is stored (e.g. on cloud memory and/or on device memory) for analysis, for example, for determining of future treatment schemes.

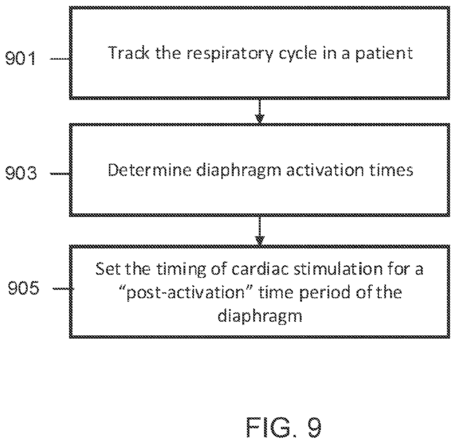

**FIG. 9** is a flowchart of a method for timing cardiac stimulation based on an excitatory state and/or contraction level of the diaphragm, according to some embodiments.

In some embodiments, the respiratory cycle of a patient is tracked (901), for example as described herein. In some embodiments, diaphragm activation times are determined (903), for example according to the phases of the respiratory cycle.

In some embodiments, timing of cardiac stimulation (such as cardiac contractility modulation stimulation) is set for a "post activation" time period of the diaphragm. In some embodiments, the "post-activation" time period is a time period immediately following a natural neural stimulation of the diaphragm, which causes the diaphragm to contract. In some embodiments, the "post-activation" time period is a time period immediately following contraction itself, for example immediately after the peak of diaphragm contraction. ("immediately following" may include, for example, 1 msec, 2 msec, 5 msec, 10 msec or intermediate, longer or shorter time).

In some embodiments, the "post-activation" time period is a time period during which neural stimulation (such as unintentional neural stimulation caused as a byproduct of cardiac contractility modulation) does not or only least results in contraction of the diaphragm. In some embodiments, the "post-activation" time period is a time period during which the diaphragm cannot contract further, therefore a neural stimulation (such as unintentional neural stimulation caused as a byproduct of cardiac contractility modulation) does not or only least results in contraction of the diaphragm.

In some embodiments, the applying of cardiac contractility modulation is timed to be delivered during the refractory period of the heart when there is at least some overlap (in time) between the refractory period of the heart and the "post-activation" period of the diaphragm. In an example, cardiac contractility modulation is timed to be delivered at the beginning of the inhalation phase, optionally following initiation of inhalation, where the diaphragm may be least or not affected by the stimulation, thereby potentially reducing pain to the patient. The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims

## Claims

1. A system comprising:
an implantable cardiac device (303) comprising:
at least one lead (305, 307) comprising one or more electrodes for applying cardiac contractility modulation stimulation to the heart; and
circuitry (801) for controlling and activating said leads, said circuitry programmed to set at least one of timing and an intensity of the cardiac contractility modulation stimulation current according to a detected respiratory rate, the respiratory rate being a parameter indicative of respiration; wherein the timing includes one or more time intervals of a respiratory cycle during which applying of cardiac contractility modulation stimulation causes least pain or sensation to the patient.

2. The system according to claim 1, further comprising a sensor (819) configured for detecting said respiratory rate.

3. The system according to claim 2, wherein said sensor (819) is selected from the group of: an acoustic sensor, a pulse oximeter, a pneumograph, a capnograph.

4. The system according to any one of claims 1-3, comprising an intra-cardiac electrode for recording ECG; wherein said respiratory rate is determined according to the recorded ECG.

5. The system according to any one of claims 1-4, wherein said circuitry (801) comprises a controller programmed to increase a rate of cardiac contractility modulation stimulations upon a detected rise in the respiratory rate and to decrease the rate of cardiac contractility modulation stimulations upon a decrease in the respiratory rate.

6. The system according to any one of claims 1-5, wherein said device comprises at least one lead comprising one or more electrodes for applying an electrical stimulation to the diaphragm and/or to the phrenic nerve.

7. The system according to claim 6, wherein said circuitry (801) comprises a controller programmed to control parameters of the stimulation applied to the diaphragm and/or phrenic nerves, said parameters comprising timing of applying the stimulation and an intensity of the stimulation current.

8. A system comprising:
one or more sensors (819) for tracking parameters of a respiratory cycle in a patient; and
an implantable cardiac device (303) comprising:
at least one lead (305, 307) comprising one or more electrodes for applying cardiac contractility modulation stimulation to the heart;
circuitry (801) for controlling and activating said leads, said circuitry programmed to set parameters of the cardiac contractility modulation stimulation according to said parameters of the respiratory cycle tracked by said one or more sensors; and
a pulse generator controlled by said circuitry to generate cardiac contractility modulation stimulation signals to be applied during one or more selected time intervals of the respiratory cycle, as tracked by said one or more sensors.

9. The system according to claim 8, wherein said one or more sensors (819) are configured to detect parameters selected from the group of: respiration rate, relative timing of inhalation and exhalation, ECG of the heart.

10. The system according to claim 8 or claim 9, wherein said parameters of the cardiac contractility modulation stimulation include timing of the cardiac contractility modulation stimulation, an intensity of the cardiac contractility modulation stimulation current.

11. The system according to any of claims 8-10, wherein said one or more selected time intervals of the respiratory cycle include one or more time intervals in which there is a larger relative anatomical space between the heart and the diaphragm and/or a larger relative anatomical space between the heart and the phrenic nerve; and
wherein said one or more selected time intervals of the respiratory cycle include one or more time intervals in which the diaphragm is farthest from the heart and/or in which the phrenic nerve is farthest from the heart.

12. The system according to any of claims 8-11, wherein said circuitry (801) is configured to time the applying the cardiac contractility modulation stimulation during an absolute refractory period of the cardiac cycle.

13. The system according to any of claims 8-12, wherein said one or more sensors (819)
a. are selected from the group of: an acoustic sensor, a pulse oximeter, a pneumograph, a capnograph;
b. include an intra-cardiac electrode which records ECG, and wherein said determining at least one respiration parameter is based on said ECG recording.

14. The system according to any of claims 8-13, wherein said circuitry (801) is configured to perform one or more of:
a. set a current intensity of the of the cardiac contractility modulation stimulation to the highest level which does not cause pain;
b. set a rate for applying multiple cardiac contractility modulation stimulations;
c. increase the rate of cardiac contractility modulation stimulations when measuring a rise in respiration rate, and decrease the rate of cardiac contractility modulation stimulations when measuring decrease in respiration rate;
d. testing, using said one or more sensors (819), during which time interval(s) applying of the cardiac contractility modulation stimulation does not cause or only least causes sensation to the patient;
e. time the applying the cardiac contractility modulation stimulation during an inhalation period;
f. time the applying the cardiac contractility modulation stimulation during an exhalation period;
g. time the applying the cardiac contractility modulation stimulation according to ECG recording obtained by said one or more sensor; (819);
h. skip the applying of said cardiac contractility modulation stimulation during one or more cardiac cycles or during one or more respiratory cycles based on said tracked parameters of the respiratory cycle.

15. The system according to claim 8, wherein said cardiac device is implanted in a patient diagnosed with heart failure.

16. The system of claim 8, wherein said one or more selected time intervals of the respiratory cycle include one or more time intervals in which there is a larger distance between said at least one lead and the phrenic nerve.

## Patentansprüche

1. System, umfassend:
eine implantierbare Herzvorrichtung (303), die Folgendes umfasst:
mindestens eine Leitung (305, 307), die eine oder mehrere Elektroden umfasst, um eine Stimulation zur Modulation der Herzkontraktilität auf das Herz anzuwenden; und
eine Schaltung (801) zum Steuern und Aktivieren der Leitungen, wobei die Schaltung so programmiert ist, dass sie mindestens einen Zeitpunkt und eine Intensität des Stimulationsstroms zur Modulation der Herzkontraktilität entsprechend einer erfassten Atemfrequenz einstellt, wobei die Atemfrequenz ein Parameter ist, der die Atmung anzeigt;
wobei der Zeitpunkt ein oder mehrere Zeitintervalle eines Atemzyklus umfasst, während derer die Anwendung der Stimulation zur Modulation der Herzkontraktilität dem Patienten am wenigsten Schmerzen oder Empfindungen verursacht.

2. System nach Anspruch 1, das ferner einen Sensor (819) umfasst, der zur Erfassung der Atemfrequenz konfiguriert ist.

3. System nach Anspruch 2, wobei der Sensor (819) aus der folgenden Gruppe ausgewählt ist: ein akustischer Sensor, ein Pulsoximeter, ein Pneumograph, ein Kapnograph.

4. System nach einem der Ansprüche 1 bis 3, das eine intrakardiale Elektrode zur Aufzeichnung eines EKGs umfasst, wobei die Atemfrequenz anhand des aufgezeichneten EKGs bestimmt wird.

5. System nach einem der Ansprüche 1 bis 4, wobei die Schaltung (801) eine Steuervorrichtung umfasst, die so programmiert ist, dass sie die Frequenz der Stimulationen zur Modulation der Herzkontraktilität bei einem erfassten Anstieg der Atmungsfrequenz erhöht und die Frequenz der Stimulationen zur Modulation der Herzkontraktilität bei einer Abnahme der Atmungsfrequenz verringert.

6. System nach einem der Ansprüche 1 bis 5, wobei die Vorrichtung mindestens eine Leitung mit einer oder mehreren Elektroden zum Anwenden einer elektrischen Stimulation auf das Zwerchfell und/oder den Zwerchfellnerv umfasst.

7. System nach Anspruch 6, wobei die Schaltung (801) eine Steuervorrichtung umfasst, die so programmiert ist, dass sie die Parameter der an das Zwerchfell und/oder die Zwerchfellnerven angelegten Stimulation steuert, wobei die Parameter den Zeitpunkt des Anwendens der Stimulation und eine Intensität des Stimulationsstroms umfassen.

8. System, umfassend:
einen oder mehrere Sensoren (819) zum Verfolgen der Parameter eines Atmungszyklus bei einem Patienten; und eine implantierbare Herzvorrichtung (303), die Folgendes umfasst:
mindestens eine Leitung (305, 307), die eine oder mehrere Elektroden umfasst, um eine Stimulation zur Modulation der Herzkontraktilität auf das Herz anzuwenden;
eine Schaltung (801) zum Steuern und Aktivieren der Leitungen, wobei die Schaltung so programmiert ist, dass sie die Parameter der Stimulation zur Modulation der Herzkontraktilität entsprechend den von dem einen oder den mehreren Sensoren erfassten Parametern des Atmungszyklus einstellt; und
einen Impulsgeber, der von der Schaltung gesteuert wird, um Stimulationssignale zur Modulation der Herzkontraktilität zu erzeugen, die während eines oder mehrerer ausgewählter Zeitintervalle des Atmungszyklus angewandt werden, wie sie von dem einen oder den mehreren Sensoren erfasst werden.

9. System nach Anspruch 8, wobei der eine oder die mehreren Sensoren (819) so konfiguriert sind, dass sie Parameter erfassen, die aus der folgenden Gruppe ausgewählt sind: Atemfrequenz, relativer Zeitpunkt des Ein- und Ausatmens, EKG des Herzens.

10. System nach Anspruch 8 oder Anspruch 9, wobei die Parameter der Stimulation zur Modulation der Herzkontraktilität den Zeitpunkt der Stimulation zur Modulation der Herzkontraktilität und die Intensität des Stimulationsstroms zur Modulation der Herzkontraktilität umfassen.

11. System nach einem der Ansprüche 8 bis 10, wobei das eine oder die mehreren ausgewählten Zeitintervalle des Atmungszyklus ein oder mehrere Zeitintervalle umfassen, in denen ein größerer relativer anatomischer Abstand zwischen dem Herzen und dem Zwerchfell und/oder ein größerer relativer anatomischer Abstand zwischen dem Herzen und dem Zwerchfellnerv besteht; und wobei das eine oder die mehreren ausgewählten Zeitintervalle des Atmungszyklus ein oder mehrere Zeitintervalle umfassen, in denen das Zwerchfell am weitesten vom Herzen entfernt ist und/oder in denen der Zwerchfellnerv am weitesten vom Herzen entfernt ist.

12. System nach einem der Ansprüche 8 bis 11, wobei die Schaltung (801) so konfiguriert ist, dass sie den Zeitpunkt der Anwendung der Stimulation zur Modulation der Herzkontraktilität während einer absoluten Refraktärzeit des Herzzyklus zeitlich misst.

13. System nach einem der Ansprüche 8 bis 12, wobei der eine oder die mehreren Sensoren (819) aus der folgenden Gruppe ausgewählt sind:
a. ein akustischer Sensor, ein Pulsoximeter, ein Pneumograph, ein Kapnograph;
b. eine intrakardiale Elektrode umfassen, die ein EKG aufzeichnet, und wobei die Bestimmung mindestens eines Atmungsparameters auf der EKG-Aufzeichnung beruht.

14. System nach einem der Ansprüche 8 bis 13, wobei die Schaltung (801) so konfiguriert ist, dass sie einen oder mehrere der folgenden Schritte ausführt:
a. Einstellen der Stromstärke der Stimulation zur Modulation der Herzkontraktilität auf die höchste Stufe, die keine Schmerzen verursacht;
b. Festlegen einer Frequenz für die Anwendung mehrerer Stimulationen zur Modulation der Herzkontraktilität;
c. Erhöhen der Frequenz der Stimulationen zur Modulation der Herzkontraktilität, wenn ein Anstieg der Atemfrequenz gemessen wird, und Verringern der Frequenz der Stimulationen zur Modulation der Herzkontraktilität, wenn ein Rückgang der Atemfrequenz gemessen wird;
d. Prüfen, unter Verwendung des einen oder der mehreren Sensoren (819), in welchem Zeitintervall bzw. in welchen Zeitintervallen die Anwendung der Stimulation zur Modulation der Herzkontraktilität keine oder nur eine geringe Empfindung bei dem Patienten verursacht;
e. Messen des Zeitpunkts der Anwendung der Stimulation der Herzkontraktilität während einer Einatmungsphase;
f. Messen des Zeitpunkts der Anwendung der Stimulation der Herzkontraktilität während einer Ausatmungsphase;
g. Messen des Zeitpunkts der Anwendung der Stimulation zur Modulation der Herzkontraktilität entsprechend der EKG-Aufzeichnung, die von dem einen oder den mehreren Sensoren (819) erhalten wird;
h. Überspringen der Anwendung der Stimulation zur Modulation der Herzkontraktilität während eines oder mehrerer Herzzyklen oder während eines oder mehrerer Atemzyklen auf der Grundlage der erfassten Parameter des Atemzyklus.

15. System nach Anspruch 8, wobei die Herzvorrichtung einem Patienten implantiert wird, bei dem eine Herzinsuffizienz diagnostiziert wurde.

16. System nach Anspruch 8, wobei das eine oder die mehreren ausgewählten Zeitintervalle des Atemzyklus ein oder mehrere Zeitintervalle umfassen, in denen ein größerer Abstand zwischen der mindestens einen Leitung und dem Zwerchfellnerv besteht.

## Revendications

1. Système comprenant :
un dispositif cardiaque implantable (303) comprenant :
au moins un conducteur (305, 307) comprenant une ou plusieurs électrodes pour appliquer au coeur une stimulation de modulation de la contractilité cardiaque ; et
un circuit (801) pour contrôler et activer les conducteurs, ce circuit étant programmé pour régler au moins l'une des durées et l'intensité du courant de stimulation de la modulation de la contractilité cardiaque en fonction d'une fréquence respiratoire détectée, la fréquence respiratoire étant un paramètre indiquant la respiration ; la durée comprenant un ou plusieurs intervalles de temps d'un cycle respiratoire pendant lequel l'application de la stimulation de modulation de la contractilité cardiaque provoque le moins de douleur ou de sensation pour le patient.

2. Système selon la revendication 1, comprenant en outre un capteur (819) configuré pour détecter la fréquence respiratoire.

3. Système selon la revendication 2, dans lequel le capteur (819) est sélectionné dans le groupe suivant : un capteur acoustique, un oxymètre de pouls, un pneumographe, un capnographe.

4. Système selon l'une des revendications 1 à 3, comprenant une électrode intra-cardiaque pour l'enregistrement de l'ECG ; dans lequel la fréquence respiratoire est déterminée en fonction de l'ECG enregistré.

5. Système selon l'une des revendications 1 à 4, dans lequel le circuit (801) comprend un contrôleur programmé pour augmenter la fréquence des stimulations de modulation de la contractilité cardiaque en cas de détection d'une augmentation de la fréquence respiratoire et pour diminuer la fréquence des stimulations de modulation de la contractilité cardiaque en cas de diminution de la fréquence respiratoire.

6. Système selon l'une des revendications 1 à 5, le dispositif comprenant au moins un conducteur comprenant une ou plusieurs électrodes pour appliquer une stimulation électrique au diaphragme et/ou au nerf phrénique.

7. Système selon la revendication 6, le circuit (801) comprenant un contrôleur programmé pour contrôler les paramètres de la stimulation appliquée au diaphragme et/ou aux nerfs phréniques, ces paramètres comprenant le moment de l'application de la stimulation et l'intensité du courant de stimulation.

8. Système comprenant :
un ou plusieurs capteurs (819) pour surveiller les paramètres d'un cycle respiratoire chez un patient ; et un dispositif cardiaque implantable (303) comprenant :
au moins un conducteur (305, 307) comprenant une ou plusieurs électrodes pour appliquer au coeur une stimulation de modulation de la contractilité cardiaque ;
un circuit (801) pour contrôler et activer les conducteurs, le circuit étant programmé pour régler les paramètres de la stimulation de modulation de la contractilité cardiaque en fonction des paramètres du cycle respiratoire mesurés par un ou plusieurs capteurs ; et
un générateur d'impulsions commandé par le circuit pour générer des signaux de stimulation de modulation de la contractilité cardiaque à appliquer pendant un ou plusieurs intervalles de temps sélectionnés du cycle respiratoire, tels qu'ils sont suivis par un ou plusieurs capteurs.

9. Système selon la revendication 8, un ou plusieurs capteurs (819) étant configurés pour détecter des paramètres sélectionnés dans le groupe suivant : fréquence respiratoire, moment relatif de l'inspiration et de l'expiration, ECG du coeur.

10. Système selon la revendication 8 ou la revendication 9, les paramètres de la stimulation de modulation de la contractilité cardiaque comprenant le moment de la stimulation de modulation de la contractilité cardiaque et l'intensité du courant de stimulation de modulation de la contractilité cardiaque.

11. Système selon l'une des revendications 8 à 10, dans lequel un ou plusieurs intervalles de temps sélectionnés du cycle respiratoire comprennent un ou plusieurs intervalles de temps dans lesquels il y a un espace anatomique relatif plus grand entre le coeur et le diaphragme et/ou un espace anatomique relatif plus grand entre le coeur et le nerf phrénique ; et dans lequel un ou plusieurs intervalles de temps sélectionnés du cycle respiratoire comprennent un ou plusieurs intervalles de temps dans lesquels le diaphragme est le plus éloigné du coeur et/ou dans lesquels le nerf phrénique est le plus éloigné du coeur.

12. Système selon l'une des revendications 8 à 11, dans lequel le circuit (801) est configuré pour chronométrer l'application de la stimulation de modulation de la contractilité cardiaque pendant une période réfractaire absolue du cycle cardiaque.

13. Système selon l'une des revendications 8 à 12, dans lequel un ou plusieurs capteurs (819) sont sélectionnés dans le groupe suivant :
a. un capteur acoustique, un oxymètre de pouls, un pneumographe, un capnographe ;
b. comprenant une électrode intra-cardiaque qui enregistre l'ECG, et la détermination d'au moins un paramètre respiratoire étant basée sur l'enregistrement de l'ECG.

14. Système selon l'une des revendications 8 à 13, le circuit (801) étant configuré pour effectuer une ou plusieurs opérations parmi les suivantes :
a. régler l'intensité du courant de la stimulation de modulation de la contractilité cardiaque au niveau le plus élevé ne provoquant pas de douleur ;
b. définir une fréquence pour l'application de multiples stimulations de modulation de la contractilité cardiaque ;
c. augmenter la fréquence des stimulations de modulation de la contractilité cardiaque lors de la mesure d'une augmentation de la fréquence respiratoire, et diminuer la fréquence des stimulations de modulation de la contractilité cardiaque lors de la mesure d'une diminution de la fréquence respiratoire ;
d. vérifier, à l'aide d'un ou plusieurs capteurs (819), pendant quel(s) intervalle(s) de temps l'application de la stimulation de modulation de la contractilité cardiaque ne provoque pas ou provoque moins de sensation chez le patient ;
e. chronométrer l'application de la stimulation de modulation de la contractilité cardiaque au cours d'une période d'inhalation ;
f. chronométrer l'application de la stimulation de modulation de la contractilité cardiaque au cours d'une période d'expiration ;
g. chronométrer l'application de la stimulation de modulation de la contractilité cardiaque en fonction de l'enregistrement ECG obtenu par le ou les capteurs (819) ;
h. sauter l'application de ladite stimulation de modulation de la contractilité cardiaque pendant un ou plusieurs cycles cardiaques ou pendant un ou plusieurs cycles respiratoires sur la base des paramètres surveillés du cycle respiratoire.

15. Système selon la revendication 8, dans lequel le dispositif cardiaque est implanté chez un patient souffrant d'insuffisance cardiaque.

16. Système selon la revendication 8, dans lequel un ou plusieurs intervalles de temps sélectionnés du cycle respiratoire comprennent un ou plusieurs intervalles de temps dans lesquels il y a une plus grande distance entre au moins une sonde et le nerf phrénique.
